(19) Europäisches Patentamt / European Patent Office / Office européen des brevets

(11) **EP 3 922 173 A1**

(12) **EUROPEAN PATENT APPLICATION**

(43) Date of publication:
**15.12.2021 Bulletin 2021/50**

(21) Application number: **20179812.1**

(22) Date of filing: **12.06.2020**

(51) Int Cl.:
*A61B 5/02* (2006.01)        *A61B 5/021* (2006.01)
*A61B 5/0285* (2006.01)      *A61B 5/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **FRACKOWIAK, Bruno Jean François**
  **5656 AE Eindhoven (NL)**
• **VAN DER HORST, Arjen**
  **5656 AE Eindhoven (NL)**

(74) Representative: **Philips Intellectual Property & Standards**
**High Tech Campus 52**
**5656 AG Eindhoven (NL)**

(54) **SYSTEMS AND METHODS FOR OBTAINING A PULSE WAVE VELOCITY MEASUREMENT**

(57)    The invention provides an apparatus for obtaining a pulse wave velocity measurement from a vessel of a subject. The apparatus is adapted to obtain measurement data from a vessel of a subject and an initial pulse wave velocity value. The measurement data is then separated into a forward wave component and a backward wave component, wherein the backward and forward wave components are associated with waves which propagate along the vessel in opposite directions. A final pulse wave velocity is then calculated by either: calculating a forward pulse wave velocity based on the forward wave component and determining a final pulse wave velocity measurement based on the forward pulse wave velocity; or calculating a backward pulse wave velocity based on the backward wave component and determining a final pulse wave velocity measurement based on the backward pulse wave velocity.

FIG. 1

**Description**

FIELD OF THE INVENTION

The invention relates to the field of vascular assessment, and more specifically to the field of pulse wave velocity measurement.

BACKGROUND OF THE INVENTION

Arterial stiffening is an important risk factor for cardiovascular disease. Arterial stiffness increases with aging and various disease states, including hypertension, hypercholesterolemia, diabetes mellitus, obesity, smoking, and kidney disease.

A commonly used parameter to assess the stiffness of vessel is pulse wave velocity (PWV). PWV is the transmission speed of pressure/flow waves (e.g. generated by the beating heart) through the arteries. The PWV is determined by the ability of the vessel to expand (i.e. distensibility, D) according to the following relation:

$$PWV = \sqrt{\frac{1}{\rho D}} \text{, with} \qquad D = \frac{dV}{VdP} \qquad (1)$$

With V the vascular volume, P the pressure, and p the blood density. From this relation, the Moens-Korteweg equation can be derived:

$$PWV = \sqrt{\frac{E \cdot h}{d \cdot \rho}} \qquad (2)$$

Here, E is the Young's modulus, d is the vessel diameter, and h is the wall thickness. By assessing the PWV, the stiffness of arteries can therefore be quantified. A typical value of the PWV in an artery is 10 m/s, which is an order of magnitude higher than the velocity of the blood particles.

The relevance of the local PWV in arteries for guiding treatment is clear from many recent studies. For example, the PWV inside the main renal artery pre-treatment is predictive of the outcome of renal denervation in patients with resistive hypertension. Low coronary PWV has also been associated with acute coronary syndromes, indicating a relation between plaque vulnerability and arterial stiffness. PWV measurements are a useful metric to obtain in any vasculature of the subject.

As the waves travel very quickly through the vessels, most commonly the PWV is determined over relatively large distances in the vasculature, e.g. brachial artery to ankle. In this way, the average stiffness of the vessels in between the measurement locations is determined.

In the last decade, more local PWV measurements have been developed. For example, for PWV assessment in both the coronary and renal anatomy, the measurement location for PWV is close to wave reflection points, which affects the accuracy of the PWV measurement.

There are multiple ways to determine PWV, the most common method being determining the delay in arrival time between pulses from one measurement location to another measurement location. This method works best for long vessels, such as the aorta as the PWV is relatively high in arteries (e.g. 6 - 20 m/s) and therefore the distance should be large to accurately measure the time delay. The measured PWV value is then a single value, spatial average PWV, over a relatively long length.

PWV measurements can vary strongly along the length of even a homogenous vessel due to the effect of wave reflections from bifurcations, stenoses or other structures with non-ideal impedance matching. The reflected waves influence the measured PWV. In long, straight vessels, such as the aorta, these deviations from the measured PWV and the reflection free PWV (having reduced, or no, wave reflection based artifacts and therefore close to the theoretical relationship in equation 1 above) are small, but if the PWV measurement is performed in close proximity to a strong reflection point, the reflection signals will significantly affect the measured PWV as the backward wave arrives at the measurements site within milliseconds.

This is typically the case for short vessels, such as the renal arteries where there are strong reflection points (for example, the bifurcation at the end of the artery) close to the PWV measurement point. In these vessels, the measured PWV cannot be used directly to assess the elasticity of the vessel via the PWV relations due to the inaccuracies caused by the reflection signals.

PWV measurements in the renal arteries are typically used to determine whether a subject with hypertension is likely to respond to renal denervation (RDN) treatment. A PWV larger than approximately 10 m/s is an indication that the subject is likely to respond to RDN treatment. As the main branch of the renal arteries are relatively short (between 5 - 8 cm), the measured PWV will be influenced by the reflections and therefore artificially higher than without any reflections. This effect is stronger when the measurement points are closer to the bifurcation.

There is therefore a need for a means of obtaining an accurate PWV measurement from vessel of a subject, and in particular form a vessel of a subject with high wave reflectivity.

SUMMARY OF THE INVENTION

The invention is defined by the claims.

According to examples in accordance with an aspect of the invention, there is provided a processor for obtaining a pulse wave velocity measurement relating to a vessel of a subject, wherein the processor is adapted to:

obtain measurement data from a vessel of a subject;
obtain an initial pulse wave velocity value for the vessel;
separate a forward wave component and/or a backward wave component of the measurement data, wherein the forward and backward wave components are associated with wave propagation along the vessel in opposite directions; and
determine a final pulse wave velocity based on:

calculating a forward pulse wave velocity based on the forward wave component and determining a final pulse wave velocity measurement based on the forward pulse wave velocity; and/or
calculating a backward pulse wave velocity based on the backward wave component and determining a final pulse wave velocity measurement based on the backward pulse wave velocity.

The processor provides for a means of obtaining a pulse wave velocity (PWV) measurement having reduced, or no wave reflection based artifacts, particularly when the measurement data is acquired from a vessel having a short length or a large number of reflection points, such as bifurcations.

A PWV measurement derived directly from a measurement obtained from the vessel of a subject will contain all of the information signals from that vessel, including reflection signals, which reduce the accuracy of the PWV measurement. In short vessels, or vessels with a large number of reflection points, the reflection signals can significantly affect the accuracy of the PWV measurement and so prevent a useful metric of that vessel from being obtained.

The signals obtained from a vessel may be separated into a forward wave component, which is associated with wave traveling away from the measurement point along the length of the vessel, and a backward wave component, which is associated with wave traveling towards the measurement point along the length of the vessel. The reflection signals are typically contained in the backward component.

By calculating the PWV based on only one of the forward or backward wave components, the influence of the convolution of forward and backward waves present in the measurement data is minimized on the final PWV.

When calculating the PWVs based on the forward and backward wave components, PWVs are calculated that are associated to the forward and backward waves thus additionally besides the influence of the convolution, also a potential deviation between two PWVs provides additional information to the user on the accuracy of the final PWV. In an embodiment the average of the backward pulse wave velocity and forward pulse wave velocity is the final PWV.

In an embodiment, when determining the final pulse wave velocity measurement based on the forward pulse wave velocity, the processor is adapted to:

compare the forward pulse wave velocity and the initial pulse wave velocity;
if the difference between the forward pulse wave velocity and the initial pulse wave velocity is greater than a predetermined threshold value, identify the forward pulse wave velocity as a new initial pulse wave velocity and return to the step of separating the initial pulse wave velocity measurement into a forward wave component and a backward wave component, wherein the initial pulse wave velocity measurement is substituted with the new initial pulse wave velocity; and
if the difference between the forward pulse wave velocity and the initial pulse wave velocity is less than or equal to a predetermined threshold value, identify the forward pulse wave velocity as the final pulse wave velocity.

By taking only the forward wave component of the measurement data in order to derive a new PWV value in an iterative manner until convergence is reached, a PWV measurement with reduced, or no, reflection signals may be obtained.

In an embodiment, when determining the final pulse wave velocity based on the backward pulse wave velocity, the processor is adapted to:

compare the backward pulse wave velocity and the initial pulse wave velocity;

if the difference between the backward pulse wave velocity and the initial pulse wave velocity is greater than a predetermined threshold value, identify the backward pulse wave velocity as a new initial pulse wave velocity and return to the step of separating the measurement data into a forward wave component and a backward wave component, wherein the initial pulse wave velocity measurement is substituted with the new initial pulse wave velocity; and

if the difference between the backward pulse wave velocity and the initial pulse wave velocity is less than or equal to a predetermined threshold value, identify the backward pulse wave velocity as the final pulse wave velocity.

In an embodiment, when obtaining the measurement data, the processor is adapted to:

obtain a first blood flow velocity and a first blood pressure acquired from a first measurement location and a second blood flow velocity and obtaining a second blood pressure acquired from a second measurement location, wherein the first measurement location and the second measurement location are spatially separated; or

obtain a first blood flow velocity and a first blood pressure acquired from a first measurement location and obtaining a second blood flow velocity acquired from a second measurement location, wherein the first measurement location and the second measurement location are spatially separated; or

obtain a first blood flow velocity and a first vessel cross-sectional area acquired from a first measurement location and obtaining a second blood flow velocity and a second vessel cross-sectional area acquired from a second measurement location, wherein the first measurement location and the second measurement location are spatially separated; or

obtain a first blood flow velocity and a first vessel cross-sectional area acquired from a first measurement location and obtain a second blood flow velocity acquired from a second measurement location, wherein the first measurement location and the second measurement location are spatially separated.

In this way, a variety of different measurements and measurement techniques, may be used to obtain a reflection-free PWV measurement. Accordingly, the most appropriate measurement technique may be selected according to the given measurement area. For example, one measurement technique may be suitable for renal PWV measurements but not coronary PWV measurements, which are both areas that suffer from high reflection signal interference.

In a further embodiment, when obtaining the blood flow velocity measurement, the processor is adapted to:

obtain a maximal blood flow velocity acquired from the center of the vessel; and

convert the maximal blood flow velocity into a cross-section average blood flow velocity.

Typically, blood flow velocity measurements are obtained from the center of a vessel; whereas, the derivation of a PWV measurement from velocity measurements typically assumes that the blood flow velocity is uniform across the vessel cross section. By converting from the measured maximal velocity to a uniform cross-sectional velocity, the coherence between the measured data and the PWV measurement derivation is improved, thereby increasing the accuracy of the final PWV measurement value.

In an embodiment, the processor is further adapted to synchronize the measurements acquired from the first measurement location and the second measuring location, wherein the synchronization is based on one or more of:

an ECG signal acquired from the subject; and

a pressure signal acquired from the subject.

In this way, the measurements may be synchronized in time, thereby increasing the accuracy of the PWV measurement.

In an embodiment, separating the forward wave component and/or the backward wave component from the measurement data is based on wave front analysis. In some additional or alternative embodiments separating the forward wave component and/or the backward wave component from the measurement data is based on the initial pulse wave velocity.

In this way, the forward and backward wave components may be accurately separated using the initial pulse wave velocity measurement.

In an embodiment, the processor is further adapted to calculate an impedance matching metric of the vessel based on a comparison between the forward wave component and the backward wave component.

In this way, in addition to obtaining a reflection-free PWV measurement, the method may also obtain a measure of the severity of the reflection points in the vessel based on the impedance.

In an embodiment, when calculating the forward pulse wave velocity based on the forward wave component or calculating the backward pulse wave velocity based on the backward wave component, the processor is adapted to perform a time-shift method applied to the forward wave component or the backward wave component.

The forward wave component and the backward wave component may be associated with a pressure wave and/or a velocity wave and/or a change in vessel cross-sectional area over time.

According to examples in accordance with an aspect of the invention, there is provided a system for obtaining a pulse wave velocity measurement from a vessel of a subject, the system comprising:

> a processor as described above; and
> a display in communication with the processor and adapted to display the pulse wave velocity to a user.

In an embodiment, the system further comprises one or more of:

> a catheter comprising one or more of:

>> a pressure sensor;
>> a velocity sensor;
>> a visual sensor;
>> a distance sensor;
>> an ultrasound transducer; and
>> a guidewire for performing a pull-back function;

> an ultrasound probe;
> an ultrasound imaging system; and
> an X-ray imaging system.

According to examples in accordance with an aspect of the invention, there is provided a method of obtaining a pulse wave velocity measurement relating to a vessel of a subject, the method comprising:

> obtaining measurement data from a vessel of a subject;
> obtaining an initial pulse wave velocity value for the vessel;
> separating a forward wave component and/or a backward wave component from the measurement data, wherein the forward and backward wave components are associated with wave propagation along the vessel in opposite directions; and
> determining a final pulse wave velocity based on one or more of:

>> calculating a forward pulse wave velocity based on the forward wave component and determining a final pulse wave velocity measurement based on the forward pulse wave velocity; and
>> calculating a backward pulse wave velocity based on the backward wave component and determining a final pulse wave velocity measurement based on the backward pulse wave velocity.

In an embodiment, determining a final pulse wave velocity measurement based on the forward pulse wave velocity comprises:

> comparing the forward pulse wave velocity and the initial pulse wave velocity;
> if the difference between the forward pulse wave velocity and the initial pulse wave velocity is greater than a predetermined threshold value, identifying the forward pulse wave velocity as a new initial pulse wave velocity and returning to the step of separating the measurement data into a forward wave component and a backward wave component, wherein the initial pulse wave velocity measurement is substituted with the new initial pulse wave velocity; and
> if the difference between the forward pulse wave velocity and the initial pulse wave velocity is less than or equal to a predetermined threshold value, identifying the forward pulse wave velocity as the final pulse wave velocity; or
> determining a final pulse wave velocity measurement based on the backward pulse wave velocity comprises:

>> comparing the backward pulse wave velocity and the initial pulse wave velocity;

if the difference between the backward pulse wave velocity and the initial pulse wave velocity is greater than a predetermined threshold value, identifying the backward pulse wave velocity as a new initial pulse wave velocity and return to the step of separating the measurement data into a forward wave component and a backward wave component, wherein the initial pulse wave velocity measurement is substituted with the new initial pulse wave velocity; and

if the difference between the backward pulse wave velocity and the initial pulse wave velocity is less than or equal to a predetermined threshold value, identifying the backward pulse wave velocity as the final pulse wave velocity.

According to examples in accordance with an aspect of the invention, there is provided a computer program product comprising computer program code means which, when executed on a computing device having a processing system, cause the processing system to perform all of the steps of the methods described above.

These and other aspects of the invention will be apparent from and elucidated with reference to the embodiments) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a method for obtaining a pulse wave velocity measurement from a vessel of a subject;
Figure 2 shows a series of graphs illustrating the pressure waves and the pulse wave velocity variation over the length of the vessel during the method of Figure 1;
Figure 3 shows a schematic representation of a typical vasculature used as an input for the numerical model of a vessel;
Figure 4 shows a schematic representation of an example of a measurement system according to an aspect of the invention;
Figure 5 shows a schematic representation of a further example of a measurement system according to an aspect of the invention; and
Figure 6 shows a schematic representation of a further example of a measurement system according to an aspect of the invention.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an apparatus for obtaining a pulse wave velocity measurement for a vessel of a subject. The apparatus is adapted to obtain measurement data from a vessel of a subject and an initial pulse wave velocity value. The measurement data is then separated into a forward wave component and/or a backward wave component, which may be based on the initial pulse wave velocity value and/or wave front analysis, wherein the backward and forward wave components are with a wave propagating along the vessel in opposite directions. A final pulse wave velocity is then calculated by either: calculating a forward pulse wave velocity based on the forward wave component and determining a final pulse wave velocity measurement based on the forward pulse wave velocity; or calculating a backward pulse wave velocity based on the backward wave component and determining a final pulse wave velocity measurement based on the backward pulse wave velocity.

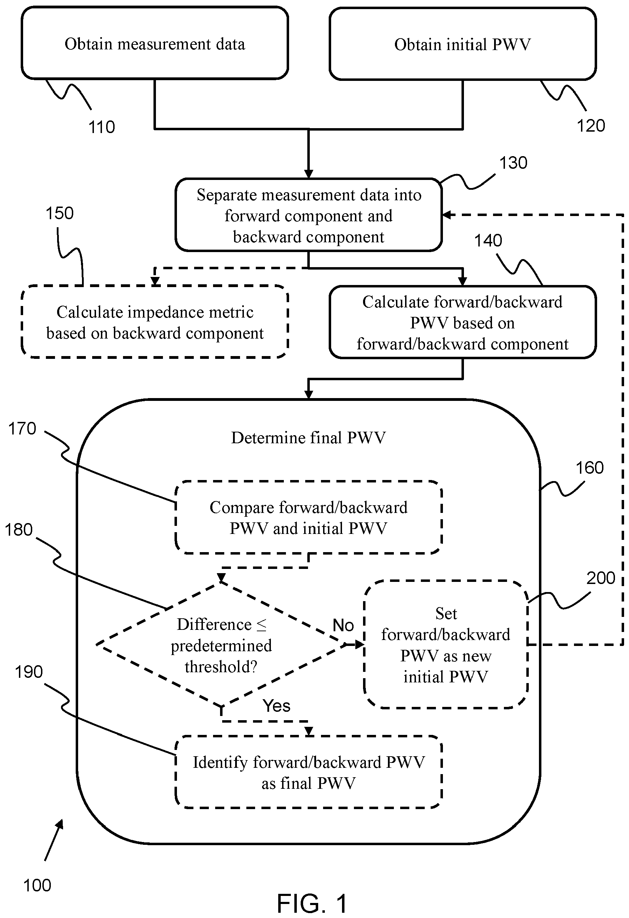

Figure 1 shows a method 100 for obtaining a pulse wave velocity measurement from a vessel of a subject.

The method begins in step 110 by obtaining measurement data from a vessel of a subject. The measurement data may be based on any suitable measurement that may be obtained from a vessel of the subject, such as blood pressure and/or blood flow velocity and/or a vessel cross sectional area. The measurement data may be obtained from a plurality of different measurement locations. For example, the measurement data may comprise a first measurement value obtained from a first measurement location and a second measurement value obtained from a second measurement location, wherein the first measurement location and the second measurement location are spatially separated. Further, measurement values of the measurement data may be obtained from more than two measurement locations in order to increase the accuracy of the PWV measurement.

For example, the measurement data may comprise a first blood flow velocity and a first blood pressure acquired from a first measurement location within the vessel of the subject and a second blood flow velocity and a second blood pressure acquired from a second measurement location within the vessel of the subject.

Alternatively, the measurement data may comprise a first blood flow velocity and a first blood pressure acquired from a first measurement location and obtaining a second blood flow velocity acquired from a second measurement location.

Alternatively, the measurement data may comprise a first blood flow velocity and a first vessel cross-sectional area acquired from a first measurement location and obtaining a second blood flow velocity and a second vessel cross-sectional area acquired from a second measurement location.

Alternatively, the measurement data may comprise a first blood flow velocity and a first vessel cross-sectional area acquired from a first measurement location and obtain a second blood flow velocity acquired from a second measurement location, wherein the first measurement location and the second measurement location are spatially separated.

Further, the measurement data may comprise any combination of blood pressure data, blood flow velocity data and vessel cross-sectional area data according to the application of the method. In particular, the measurement data may be adjusted according to the measurement equipment available.

For example, a system for obtaining the PWV measurement from a vessel of the subject may include one or more catheters comprising one or more of: a pressure sensor for obtaining blood pressure data; a velocity sensor for obtaining blood flow velocity data; a visual sensor, for example in combination with radiopaque markers, a distance sensor or an ultrasound transducer for obtaining blood flow velocity data and/or blood vessel cross-sectional area data; and a guidewire for performing a pull-back function for moving the sensors along the length of the vessel. Further, the system may comprise an ultrasound probe and/or an ultrasound imaging system, external to the vessel of the subject, adapted to obtain blood flow velocity data and/or blood vessel cross-sectional area data from the vessel of interest. The system may further comprise an X-ray imaging system to acquire images of the vessel structure, such as an angiography system.

In step 120, an initial pulse wave velocity measurement is obtained. The initial pulse wave velocity may be an initial estimate based on typical population data values related to the artery of interest or may be set to a predetermined value, such as 10 m/s.

In step 130, the measurement data is separated into a forward wave component and a backward wave component, wherein the forward wave component is associated with a wave which propagates along the vessel in one direction and the backward wave component is associated with a wave which propagates along the vessel in an opposite direction. The forward wave component and the backward wave component may be associated with a pressure wave and/or a velocity wave and/or a cross sectional wave, representing the change in vessel cross section over time.

Separating the initial measurement data into a forward wave component and a backward wave component may be based on wave front analysis, for example using a water hammer equation. Water hammer equations, also referred to as hydraulic shock equations and also known as the slope method, may be used to calculate the PWV of a vessel from simultaneous pressure (P) and flow velocity (U) measurements inside the vessel a period that is less sensitive to wave reflection (e.g. early systole):

$$PWV = \frac{1}{\rho}\frac{dP}{dU} \qquad (3)$$

Alternatively, in case this period cannot be used the following relation can be used that determines the PWV by summation over the whole cardiac cycle, under the condition of uncorrelated forward and backward waves:

$$PWV = \frac{1}{\rho}\sqrt{\frac{\sum dP^2}{\sum dU^2}} \qquad (4)$$

This slope method is described in further detail in Davies et al, 2006, "Use of simultaneous pressure and velocity measurements to estimate arterial wave speed at a single site in humans", Am J Physiol Heart Circ Physiol. 290(2):H878-85.

The PWV can be determined via Equations 3 or 4 above. Although the sampling frequency does not need to be so high, a disadvantage of calculating the PWV via Equations 3 or 4 above is the absence of a means for accounting for reflections from downstream vasculature, meaning that in cases of strong reflections the method gives highly inaccurate results.

Rather than performing a direct calculation of the PWV using the water hammer equation above, separation of measurement data into forward and/or backward wave components can be done at first instance based on the initial pulse wave velocity value, as follows.

The estimation of the forward (P+/U+) and backward waves (P-/U-) are based on the following water hammer derived equations:

$$dP_+ = \frac{1}{2}(dP + \rho c dU)$$

$$dP_- = \frac{1}{2}(dP - \rho c dU)$$

$$dU_+ = \frac{1}{2\rho c}(dP + \rho c dU)$$

$$dP_+ = \frac{1}{2\rho c}(dP - \rho c dU)$$

wherein: $\rho$ is the blood density; and c is a PWV value, which may be the initial PWV or an intermediate PWV.

The increments of pressure (dP) and velocity (dU) are determined by differentiating the initial pressure and velocity signal:

$$dP = P(t + \Delta t) - P(t)$$

$$dU = U(t + \Delta t) - U(t)$$

The forward wave component and the backward wave component may then be reconstructed by integrating the respective increments determined from the water hammer equations with respect to time as followed:

$$P\pm = \int dP\pm dt \approx \sum dP\pm$$

$$U\pm = \int dU\pm dt \approx \sum dU\pm$$

In step 140, a forward PWV value is calculated based on the forward wave component. Calculating the forward pulse wave velocity based on the forward wave component may be based on a time-shift method applied to the forward wave component. One example of a time-shift method is a foot-to-foot method. Alternatively, a peak-to-peak method or any other suitable time-shift method based on features of the waves may be used. For example, when the forward wave component is associated with a pressure wave, calculating the forward pulse wave velocity may be performed as follows.

The forward pressure waves, obtained for each of the measurement locations, may first be normalized and a vertical position may be defined on the upslope of the forward pressure wave. The vertical position may be defined as a percentage, such as 30%, between the minimum (=0) and maximum (=1) of the forward pressure wave. The percentage may be based on a tradeoff between two trends. Firstly, the estimation of the time difference $\Delta t$ is more accurate on the vertical part of the signal, referred to as the upslope, compared to the foot, or minimum, of the signal. Even if the line falls between two discrete points, the interpolation error in terms of time will be lowered if the line is vertical because the time difference between the two sampled discrete points is smaller. Secondly, the reflected wave affects the signal more on the diastolic part compared to the systolic upslope, meaning the closer the position of the sample point to the foot, or minimum, of the signal, the lower the influence from the reflected wave. This affects the accuracy of decomposing the signal into forward and backward wave components, and hence the accuracy of the iterative process of determining the final PWV.

With the vertical position fixed, the time difference Δt between two curves (the two curves representing the forward pressure wave at the first measurement location and the second measurement location) may be determined. Interpolation may be performed if the line falls in-between two discrete points. Using the time difference Δt between the two curves and the distance dx between the respective measurement points, the pulse wave velocity may be deduced as:

$$PWV = \frac{\Delta x}{\Delta t}$$

In addition to calculating the forward PWV value based on the forward wave component, the backward PWV may be calculated based on the backward wave component as described above. Further, the method may include the further step 150 of calculating an impedance matching metric of the vessel based on the backward wave component.

As the backward wave component includes the reflection signals, it is possible to determine the impedance matching at the reflection points along the vessel. The impedance matching may be indirectly determined by comparing the backward wave component to the forward wave component. In an ideal case of perfect impedance matching, the backward wave component would be equal to 0, whereas the forward wave component would be equal to the total wave. In the case of a maximal mismatch, the wave would be fully reflected, meaning the forward wave component would be equal to the backward wave component. A comparison of the maximal respective values of forward and backward wave components may be used to assess the degree of impedance mismatch in the vessel of interest. Additional post processing may be implemented, for example, by mapping the full signal shape from the vessel.

In step 160, a final pulse wave velocity is determined based on the forward pulse wave velocity or the backward pulse wave velocity. The final pulse wave velocity may be determined from the forward pulse wave velocity or the backward pulse wave velocity using any suitable method.

For example, determining a final pulse wave velocity measurement based on the forward pulse wave velocity or the backward pulse wave velocity may include the step 170 of comparing the forward pulse wave velocity or the backward pulse wave velocity to the initial pulse wave velocity.

It may then be determined in step 180 if the difference between the forward pulse wave velocity or the backward pulse wave velocity and the initial pulse wave velocity is less than or equal to a predetermined threshold value.

If the difference between the forward pulse wave velocity or the backward pulse wave velocity and the initial pulse wave velocity is less than or equal to a predetermined threshold value, the method may proceed to step 190 and identify the forward pulse velocity or the backward pulse wave velocity as the final pulse wave velocity.

If the difference between the forward pulse wave velocity or the backward pulse wave velocity and the initial pulse wave velocity is greater than a predetermined threshold value, the method may proceed to step 200 and identifying the forward pulse wave velocity or the backward pulse wave velocity as a new initial pulse wave velocity and returning to step 130, wherein the initial pulse wave velocity is substituted with the new initial pulse wave velocity.

Put another way, a new PWV may be derived from the forward wave component or the backward wave component of the previous PWV measurement in an iterative manner until the two measurements converge. As the PWV value is always calculated based on the forward wave component or the backward pulse wave velocity, each iteration of the PWV value will contain fewer reflection signals, thereby converging on a true PWV value.

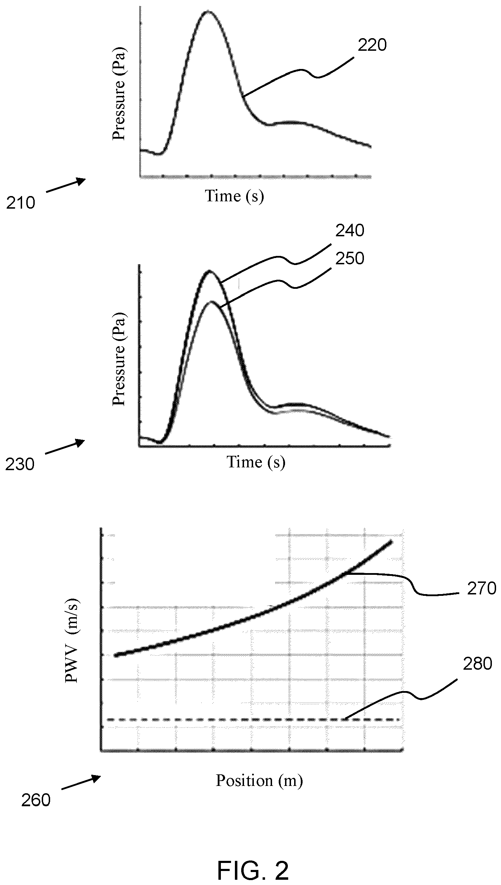

Figure 2 shows a series of graphs illustrating the pressure waves during the method of Figure 1 described above.

The first graph 210 is a graph of pressure against time and the plot line 220 represents a pressure wave obtained from a vessel of the subject. The second graph 230 is a graph of pressure against time and includes a plot line 240 representing the forward wave component of the pressure wave 220 and a plot line 250 representing the backward wave component of the pressure wave 220.

The third graph 260 is a graph of PWV against position in the vessel and includes a plot line 270 representing the PWV obtained directly from the pressure wave 220 without performing the steps of the method described above with reference to Figure 1. In addition, graph 260 shows a plot line 280 representing the reflection-free PWV obtained from the forward or backward wave components as described about with reference to Figure 1. As can be seen from graph 260, there is a significant difference between the PWV value obtained from the full pressure signal and the reflection-free PWV value obtained from the forward or backward wave components.

The methods described above have been validated in simulations using a 1D vasculature model and bench experiments. The methods described above may be applied to any vessel of interest. In an example, 1D vasculature model may include some of the renal vasculature, whose dimensions may be estimated from an angiography image, and calculates the PWV in the main branch. The 1D vasculature model may be simplified to include only a single vessel coupled to a 0D Windkessel boundary condition, mimicking the flow resistance from the downstream vasculature and the impedance mismatch at the first bifurcation. For both model configurations, a parametrical study is carried out, either by changing the vessel stiffness and the wall thickness of the main branch, or by changing the Windkessel compliance and resistance to mimic different levels of vasoconstriction.

The inventors have found that the PWV estimated from the forward propagating pressure component P+ is very close to an idealized reflection-free PWV, compared to the PWV estimated from the initial pressure signal.

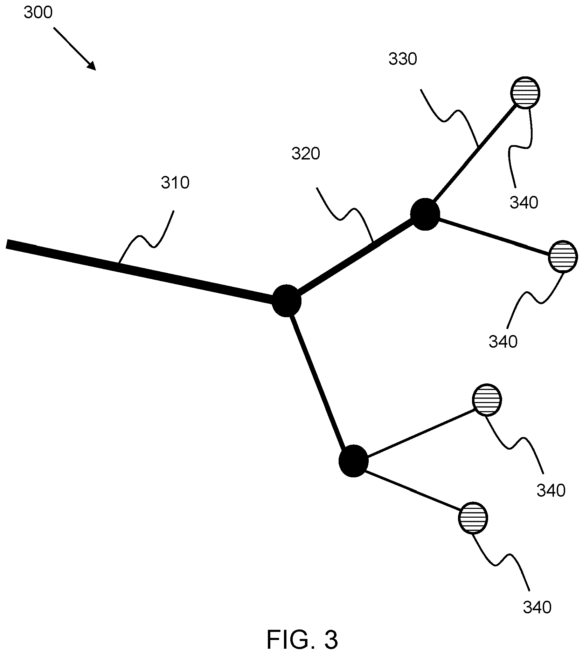

Figure 3 shows a schematic representation 300 of typical vasculature used in the 1D numerical model of the vessel, wherein the vessel comprises a main branch 310, a first primary branch 320 and a second primary branch 330. There is an outlet boundary 340, which may have an outlet boundary condition equivalent to a Windkessel 0D model, wherein the impedance of the outlet boundary is dependent on the resistance ($R_v$) and compliance ($C_v$) of the vessel, which in turn are outlet radius ($r_O$) dependent. The relationships of the model may be determined from a Structured Fractal Tree Model (SFTM) as described in Olufsen et al "Structured tree outflow condition for blood flow in larger systemic arteries", The American Physiological Society, 1999.

The SFTM is generated for resting conditions and does not take into account the vasoconstriction, for example due to the overdrive in the sympathetic nerve when considering renal vasculature. The compliance doesn't significantly influence the pulse wave velocity in the main branch of the vessel; however, even a slight change in the downstream vasculature radius can have a significant effect on the overall resistance, which influences the pulse wave velocity profile in the main branch.

Complementary to the numerical analysis with the 1D vasculature model, experimental measurements of pressure and velocity made on a bench test using a catheter comprising a pressure sensor and a velocity sensor in the form of an ultrasound transducer to obtain pressure and velocity measurements at different locations in a single tube with a known wave reflection at the outlet. A roller pump was used to generate a pulsed flow at the inlet, comparable to the physiological flow present in arteries.

By applying the methods described above with reference to Figure 1 to the experimental pressure and velocity measurements, the resulting PWV estimated based on the forward pressure component P+ was also very close to the idealized reflection-free PWV value.

It should be noted that when the blood flow velocity is measured using an ultrasound Doppler technique, the measured velocity value corresponds to the maximal value at the center of the tube, whereas the 1D vasculature model assumes a uniform velocity field across the cross section of the tube, corresponding to the average value. The Womersley number is a relevant criterion to bridge the gap between the maximal blood flow velocity and a cross-section average blood flow velocity. Indeed, the Womersley number (Wo) corresponds to the ratio between the transient inertial forces and the viscous forces, and can be expressed according to the following equation:

$$W_O = \frac{[inertial\ forces]}{[viscous\ forces]} = \frac{d}{2}\sqrt{\frac{2\pi f \rho}{\mu}}$$

wherein: p represents the blood density; $f$ is the typical frequency of the flow (roughly 1Hz for a heartbeat); $\mu$ is the dynamic viscosity of the fluid.

For pulsating flow regimes in blood vessels, the time varying radial velocity profile can be determined from the following analytical expression (with $P'_n$ representing the longitudinal pressure gradient of the harmonic $n\omega$):

$$u(r,t) = Re\left\{\sum_{n=0}^{N} \frac{i \times P'_n}{2\pi\rho} \times \left[1 - \frac{J_O\left(W_0 \times n^{\frac{1}{2}} \times i^{\frac{3}{2}} \times \frac{2r}{d}\right)}{J_O\left(W_0 \times n^{\frac{1}{2}} \times i^{\frac{3}{2}}\right)}\right] \times e^{2\pi i n f t}\right\}$$

wherein: i is the imaginary number; r is the radial coordinate; t is time; d is the vessel diameter; Re is the real part of the complex number; N is the total amount of harmonics in the discrete solution (if N tends to the infinity, the continuous solution is reached); and $J_0$ is a Bessel function of order 0.

A low Womersley number (Wo < 1) corresponds to a viscous regime where the flow variation in time is slow (low frequency), thereby allowing the full development of the flow into a parabolic radial profile, such as Poiseuille viscous flow where the average flow is ½ of the maximal measured flow. A large Womersley number (Wo > 10) corresponds to a plug flow regime, where the flow variation in time is fast (high frequency) and providing no time for the flow to develop. Hence, the flow radial profile remains flat meaning the average flow is equal to the maximal measured flow.

In practice, the Womersley number can be estimated using the diameter of the blood vessel and the frequency of the flow variation (*f*). From that number, the flow regime can be determined. The maximal blood flow velocity may then be converted to a cross-section average blood flow velocity using the equation above.

Several example of the practical application of the methods and systems described above are provided below.

In a first example, the time varying maximal blood flow velocity and blood pressure are determined at two different measurement locations within a vessel, using either a catheter having both a pressure and velocity sensor or two separate flow and pressure measurement devices and performing a pullback maneuver from one measuring location to another.

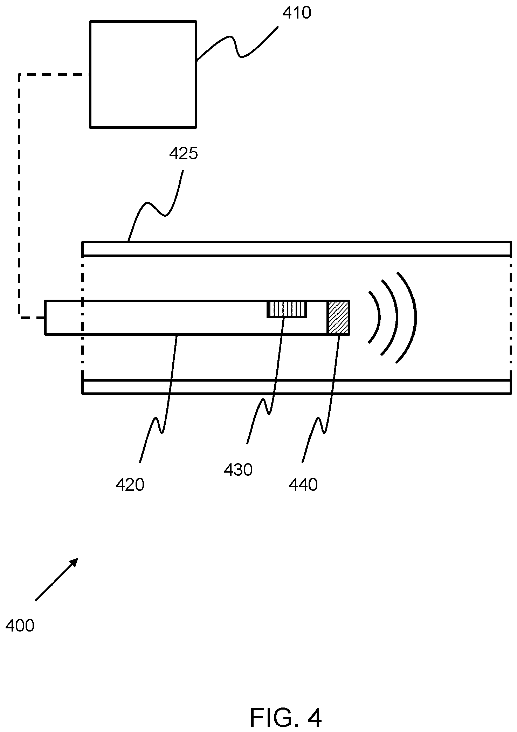

Figure 4 shows a schematic representation 400 of the first example measurement system comprising a processor 410 adapted to implement the methods described above in communication with a catheter 420 disposed within a vessel 425 of the subject. The catheter comprises a pressure sensor 430 and a velocity sensor 440 in order to obtain measurement values (which constitute the measurement data) from the vessel. The catheter may be moved from a first measurement location to a second measurement location within the vessel in order to obtain the measurement data.

Additional measurement locations may be used to improve the measurement accuracy at the expense of measurement speed. Overall, the measurements are taken at measurement locations a known distance apart and may be synchronized via an ECG signal or the pressure signal measured on the catheter sensor. Synchronization via ECG may be used where the measurement data are obtained from different locations and are not performed simultaneously, for example, when performing a pull back operation. If the measurement data can be obtained simultaneously, ECG synchronization may not be needed. The pressure signal from the catheter should be obtained at the same time as measurement data is obtained at another location to be used for synchronization. The maximal blood flow velocity may be converted into a cross-sectional average blood flow velocity as described above.

The iterative process to determine the PWV from the forward pressure wave P+ according to the method described above with reference to Figure 1 is then performed using the measurement data and the final reflection-free PWV may be displayed to the user. In addition, an estimation of the backward propagating pressure signal P- may be performed and the degree of impedance mismatch at the bifurcation may also be displayed to the user.

The pullback method provides accurate measurement values but can be cumbersome and time consuming.

In a second example, a time varying maximal blood flow velocity signal is determined at two or more measurement locations using a velocity sensor such as a flow wire, whereas the pressure signal is measured at only a single measurement location, which may be the same as one of the locations at which the blood flow velocity signal is measured or a different location, such as at the beginning of the vessel branch. ECG gating, or gating based on the pressure signal if the pressure signal is obtained simultaneously to the blood flow velocity measurements, may be used for synchronization between the velocity and pressure signals and also between the position dependent velocity signals themselves. Otherwise, the distance between the fixed pressure measurement location and the varying flow velocity measurement location may be estimated, either by knowing the pullback distance or using imaging techniques with radiopaque markers on the wires.

Unlike the first example, the PWV is determined from the forward velocity signal U+ because the time-shift method implies several measurements at different locations. On top of the reflection free PWV value, the degree of impedance mismatch at the bifurcation may be assessed from the backward propagating velocity signals.

The second example is less accurate than the first example because the pressure signal is measured at only one measurement location; however, the final PWV is still close to the value obtained from the full set of pressure and velocity values. Moreover, the second example offers more flexibility in the clinical procedure by allowing use of flow wire or flow catheter devices.

In a third example, time varying maximal blood flow velocity measurements and blood pressure measurements are determined at two or more locations using multiple flow velocity sensors and multiple pressure sensors. The processing method is the same as in the first example embodiment 1; however, instead of doing a pullback maneuver, the measurements are achieved by inserting several measurement devices in the arteries at the different measurement locations. Further, intravascular devices having several pressure and velocity sensors embedded along one wire at different locations may be used.

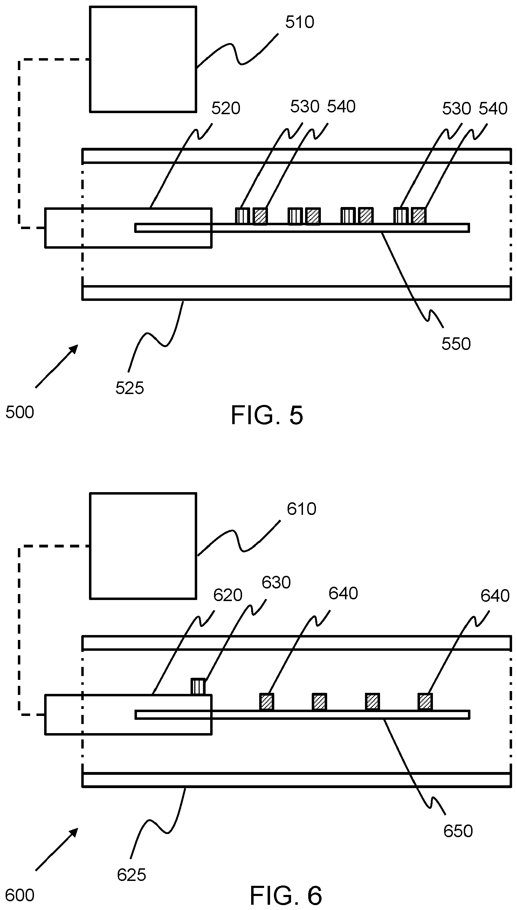

Figure 5 shows a schematic representation 500 of the third example measurement system comprising a processor 510 adapted to implement the methods described above in communication with a catheter 520 disposed within a vessel 525 of the subject. The catheter comprises a plurality of pressure sensors 530 and a plurality of velocity sensors 540 along the length of a guidewire 550 in order to obtain measurement data from the vessel.

In a fourth example, a time varying maximal blood flow velocity signal is determined at two or more measurement locations using multiple flow sensors, whereas the pressure signal is measured at a single measurement location, such as at the beginning of the vessel branch. The processing method is the same as in the second example; however, instead of doing a pullback maneuver, the flow measurements may be achieved by inserting several flow measurement devices in the vessel at the different measurement locations. Additionally, a pressure sensor may be located on a guiding catheter, on a guidewire, or on a separate pressure wire.

Figure 6 shows a schematic representation 600 of the fourth example measurement system comprising a processor 610 adapted to implement the methods described above in communication with a catheter 620 disposed within a vessel 625 of the subject. The catheter comprises a pressure sensor 630 and a plurality of velocity sensors 640 in order to obtain measurement data from the vessel.

In the third and fourth example, ECG gating is not needed for synchronizing the measurement data, thereby simplifying the processing of the measurement data.

In a fifth example, time varying maximal blood flow velocity signals are measured at two or more measurement locations using any of the methods described above. Instead of measuring the pressure signal, the time varying cross-sectional areas of the vessel may be measured at those same measurement locations, for example using an intravascular ultrasound (IVUS) device or an optical coherence tomography device.

As the vessel compliance relates to its mechanical properties, the pressure signal increment dP relates directly to the area increment dA and to the PWV value as shown in the following equations:

$$C_O = \frac{dA}{dP} = \frac{2\pi r^3}{Eh}$$

$$dP = dA \times \frac{Eh}{2\pi r^3} = dA \times \frac{\rho}{A_O} \times c^2$$

wherein: Ao is a reference area (when the vessel is in equilibrium without vessel wall motion); Co is the compliance of the vessel when $A=A_O$; E is the Young modulus - or stiffness - of the vessel; and h is the vessel wall thickness.

This relation results in a rewriting of the water hammer derived equations discussed above as follows:

$$dP_+ = \frac{1}{2}(dA \times \frac{\rho}{A_O} \times c^2 + \rho c dU)$$

$$dP_- = \frac{1}{2}(dA \times \frac{\rho}{A_O} \times c^2 - \rho c dU)$$

$$dU_+ = \frac{1}{2}(dA \times \frac{c}{A_O} + dU)$$

$$dP_+ = \frac{1}{2}(dA \times \frac{c}{A_O} - dU)$$

The algorithm for determining the forward wave component and the backward wave component for the pressure and velocity waves relies on different equations, but the overall method remains similar.

Although the cross-sectional area measurement with an IVUS catheter is a different technology than pressure measurement with a guide wire, the method remains similar to the first example with two different measurement devices being inserted inside the vessel.

In a sixth example, time varying maximal blood flow velocity signals are measured at two or more locations, whereas the time varying cross-sectional area signals are measured using extravascular ultrasound (EVUS). The processing method is the same as the fifth example except that EVUS is used instead of IVUS. The intravascular flow sensor must be visible on the EVUS image in order to determine the distance between the velocity signals and the cross-sectional area signals. In this example, only one device is inserted inside the body to obtain the time varying velocity signal.

In a seventh example, time varying maximal blood flow velocity and cross section area signals are directly estimated from the EVUS images, making the procedure completely non-intrusive. Moreover, since both signals are located at the same points on the images, synchronization by ECG gating, is not necessary. In this way, the procedure is simplified and is comparable to the first example in terms of accuracy.

In an eighth example, time varying velocity signals are directly estimated from the EVUS images at several points and a pressure signal is estimated with an intravascular device at one or more measurement locations. The intravascular pressure sensor must be visible on the EVUS images in order to the estimated velocity signals and the pressure signals.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1.    A processor (410) for obtaining a pulse wave velocity measurement relating to a vessel (425) of a subject, wherein the processor is adapted to:

obtain measurement data from the vessel of the subject;
obtain an initial pulse wave velocity value for the vessel;
separate a forward wave component and/or a backward wave component of the measurement data, wherein the forward and backward wave components are associated with wave propagation along the vessel in opposite directions; and
determine a final pulse wave velocity based on:

calculating a forward pulse wave velocity based on the forward wave component and determining a final pulse wave velocity measurement based on the forward pulse wave velocity; and/or
calculating a backward pulse wave velocity based on the backward wave component and determining a final pulse wave velocity measurement based on the backward pulse wave velocity.

2.    A processor (410) as claimed in claim 1, wherein, when determining the final pulse wave velocity based on the forward pulse wave velocity, the processor is adapted to:

compare the forward pulse wave velocity and the initial pulse wave velocity;
if the difference between the forward pulse wave velocity and the initial pulse wave velocity is greater than a predetermined threshold value, identify the forward pulse wave velocity as a new initial pulse wave velocity and return to the step of separating the measurement data into a forward wave component and a backward wave component, wherein the initial pulse wave velocity measurement is substituted with the new initial pulse wave velocity; and
if the difference between the forward pulse wave velocity and the initial pulse wave velocity is less than or equal to a predetermined threshold value, identify the forward pulse wave velocity as the final pulse wave velocity.

3.    A processor (410) as claimed in claim 1, wherein, when determining the final pulse wave velocity based on the backward pulse wave velocity, the processor is adapted to:

compare the backward pulse wave velocity and the initial pulse wave velocity;
if the difference between the backward pulse wave velocity and the initial pulse wave velocity is greater than a predetermined threshold value, identify the backward pulse wave velocity as a new initial pulse wave velocity and return to the step of separating the measurement data into a forward wave component and a backward wave component, wherein the initial pulse wave velocity measurement is substituted with the new initial pulse wave velocity; and
if the difference between the backward pulse wave velocity and the initial pulse wave velocity is less than or equal to a predetermined threshold value, identify the backward pulse wave velocity as the final pulse wave velocity.

4.    A processor (410) as claimed in any of claims 1 to 3, wherein for obtaining the measurement data, the processor is adapted to:

obtain a first blood flow velocity and a first blood pressure acquired from a first measurement location and obtain

13

a second blood flow velocity and a second blood pressure acquired from a second measurement location, wherein the first measurement location and the second measurement location are spatially separated; or obtain a first blood flow velocity and a first blood pressure acquired from a first measurement location and obtain a second blood flow velocity acquired from a second measurement location, wherein the first measurement location and the second measurement location are spatially separated;

obtain a first blood flow velocity and a first vessel cross-sectional area acquired from a first measurement location and obtain a second blood flow velocity and a second vessel cross-sectional area acquired from a second measurement location, wherein the first measurement location and the second measurement location are spatially separated; or

obtain a first blood flow velocity and a first vessel cross-sectional area acquired from a first measurement location and obtain a second blood flow velocity acquired from a second measurement location, wherein the first measurement location and the second measurement location are spatially separated.

5. A processor (410) as claimed in claim 4, wherein, when obtaining the blood flow velocity measurement, the processor is adapted to:

obtain a maximal blood flow velocity acquired from the center of the vessel; and
convert the maximal blood flow velocity into a cross-section average blood flow velocity.

6. A processor (410) as claimed in any of claims 4 to 5, wherein the processor is further adapted to synchronize the measurements acquired from the first measurement location and the second measuring location, wherein the synchronization is based on one or more of:

an ECG signal acquired from the subject; and
a pressure signal acquired from the subject.

7. A processor (410) as claimed in any of claims 1 to 6, wherein separating the forward wave component and the backward wave component of the measurement data is based on wave front analysis.

8. A processor (410) as claimed in any of claims 1 to 7, wherein the processor is further adapted to calculate an impedance matching metric of the vessel based on a comparison between the forward wave component and the backward wave component.

9. A processor (410) as claimed in any of claims 1 to 8, wherein, when calculating the forward pulse wave velocity based on the forward wave component or calculating the backward pulse wave velocity based on the backward wave component, the processor is adapted to perform a time-shift method applied to the forward wave component or the backward wave component.

10. A processor (410) as claimed in any of claims 1 to 9, wherein separating the forward wave component and the backward wave component of the measurement data is based on the initial pulse wave velocity value.

11. A system (400) for obtaining a pulse wave velocity measurement from a vessel of a subject, the system comprising:

a processor (410) as claimed in any of claims 1 to 10; and
a display in communication with the processor and adapted to display the pulse wave velocity to a user.

12. A system (400) as claimed in claim 11, wherein the system further comprises one or more of:

a catheter (420) comprising one or more of:

a pressure sensor (430);
a velocity sensor (440);
a visual sensor;
a distance sensor;
an ultrasound transducer; and
a guidewire for performing a pull-back function;

an ultrasound probe;

an ultrasound imaging system; and
an X-ray imaging system.

13. A method (100) of obtaining a pulse wave velocity measurement relating to a vessel of a subject, the method comprising:

obtaining (110) measurement data from the vessel of the subject;
obtaining (120) an initial pulse wave velocity value for the vessel;
separating a forward wave component and/or a backward wave component of the measurement data, wherein the forward and backward wave components are associated with wave propagation along the vessel in opposite directions; and
determining a final pulse wave velocity (160) based on one or more of:

calculating a forward pulse wave velocity based on the forward wave component and determining a final pulse wave velocity measurement based on the forward pulse wave velocity; and
calculating a backward pulse wave velocity based on the backward wave component and determining a final pulse wave velocity measurement based on the backward pulse wave velocity.

14. A method as claimed in claim 13, wherein:
determining a final pulse wave velocity measurement based on the forward pulse wave velocity comprises:

comparing (170) the forward pulse wave velocity and the initial pulse wave velocity;
if the difference between the forward pulse wave velocity and the initial pulse wave velocity is greater than a predetermined threshold value, identifying (200) the forward pulse wave velocity as a new initial pulse wave velocity and returning to the step of separating the measurement data into a forward wave component and a backward wave component, wherein the initial pulse wave velocity measurement is substituted with the new initial pulse wave velocity; and
if the difference between the forward pulse wave velocity and the initial pulse wave velocity is less than or equal to a predetermined threshold value, identifying (190) the forward pulse wave velocity as the final pulse wave velocity; or
determining a final pulse wave velocity measurement based on the backward pulse wave velocity comprises:

comparing (170) the backward pulse wave velocity and the initial pulse wave velocity;
if the difference between the backward pulse wave velocity and the initial pulse wave velocity is greater than a predetermined threshold value, identifying (200) the backward pulse wave velocity as a new initial pulse wave velocity and return to the step of separating the measurement data into a forward wave component and a backward wave component, wherein the initial pulse wave velocity measurement is substituted with the new initial pulse wave velocity; and
if the difference between the backward pulse wave velocity and the initial pulse wave velocity is less than or equal to a predetermined threshold value, identifying (190) the backward pulse wave velocity as the final pulse wave velocity.

15. A computer program product comprising computer program code means which, when executed on a computing device having a processing system, cause the processing system to perform the steps of the method according to claim 13 or 14.

FIG. 1

FIG. 2

300

330

320

310

340

340

340

340

FIG. 3

FIG. 4

FIG. 5

FIG. 6

Europäisches
Patentamt

European
Patent Office

Office européen
des brevets

# EUROPEAN SEARCH REPORT

**Application Number**

EP 20 17 9812

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 354 999 B1 (DGANY ELHANAN [IL] ET AL) 12 March 2002 (2002-03-12) | 1-6,9-15 | INV.<br>A61B5/02<br>A61B5/021 |
| Y | * column 4, lines 13-47 - column 5, lines 34-53 *<br>* column 11, lines 42-62 *<br>* column 13, lines 48-67 *<br>* column 17, lines 50-64 *<br>----- | 7,8 | A61B5/0285<br>A61B5/00 |
| X | US 2019/290139 A1 (SIO CHARLES FREDERIK [NL] ET AL) 26 September 2019 (2019-09-26)<br>* paragraphs [0030] - [0043], [0071] *<br>----- | 1-5,<br>10-12 | |
| X | DAIMÉ CAMPOS ARIAS ET AL: "Mapping the site-specific accuracy of loop-based local pulse wave velocity estimation and reflection magnitude: a 1D arterial network model analysis",<br>CLINICAL PHYSICS AND PHYSIOLOGICAL MEASUREMENT,<br>vol. 40, no. 7, 1 July 2019 (2019-07-01), page 075002, XP055699244,<br>GB<br>ISSN: 0143-0815, DOI: 10.1088/1361-6579/ab15aa<br>* page 3, lines 38-49 *<br>* figure 2 *<br>----- | 1,11 | |
| Y | HUGHES A D ET AL: "Forward and backward waves in the arterial system: impedance or wave intensity analysis?",<br>MEDICAL & BIOLOGICAL ENGINEERING & COMPUTING, SPRINGER, BERLIN, DE,<br>vol. 47, no. 2,<br>6 February 2009 (2009-02-06), pages 207-210, XP019835347,<br>ISSN: 1741-0444<br>* abstract *<br>----- | 7,8 | |

**TECHNICAL FIELDS SEARCHED (IPC)**

A61B

The present search report has been drawn up for all claims

| Place of search | Date of completion of the search | Examiner |
|---|---|---|
| The Hague | 11 November 2020 | Kowalczyk, Szczepan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 17 9812

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2020

| Patent document cited in search report | | Publication date | Patent family member(s) | | Publication date |
|---|---|---|---|---|---|
| US 6354999 | B1 | 12-03-2002 | NONE | | |
| US 2019290139 | A1 | 26-09-2019 | CN | 109152540 A | 04-01-2019 |
| | | | EP | 3457927 A1 | 27-03-2019 |
| | | | JP | 2019518519 A | 04-07-2019 |
| | | | US | 2019290139 A1 | 26-09-2019 |
| | | | WO | 2017198813 A1 | 23-11-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **DAVIES et al.** Use of simultaneous pressure and velocity measurements to estimate arterial wave speed at a single site in humans. *Am J Physiol Heart Circ Physiol.*, 2006, vol. 290 (2), H878-85

- **OLUFSEN et al.** Structured tree outflow condition for blood flow in larger systemic arteries. *The American Physiological Society,* 1999